(19) Europäisches Patentamt
European Patent Office
Office européen des brevets

(11) Numéro de publication: **0 371 874 A1**

(12) **DEMANDE DE BREVET EUROPEEN**

(21) Numéro de dépôt: **89403293.7**

(51) Int. Cl.[5]: **A61K 7/48, C07D 473/08**

(22) Date de dépôt: **28.11.89**

(30) Priorité: **29.11.88 FR 8815575**

(43) Date de publication de la demande: **06.06.90 Bulletin 90/23**

(84) Etats contractants désignés: **BE CH DE ES GB IT LI NL**

(71) Demandeur: **PIERRE FABRE COSMETIQUE**
**125 rue de la Faisanderie**
**F-75116 Paris(FR)**

(72) Inventeur: **Trebosc, Marie-Thérèse**
**19, rue Baron Cachin**
**F-81100 Castres(FR)**
Inventeur: **Mouzin, Gilbert**
**11, rue des Pénitents blancs**
**F-31000 Toulouse(FR)**
Inventeur: **Cousse, Henri**
**La Foun de los Nobios Chemin de Lastinos**
**F-81100 Castres(FR)**

(74) Mandataire: **Warcoin, Jacques et al**
**Cabinet Régimbeau 26, avenue Kléber**
**F-75116 Paris(FR)**

(54) **Amincissants topiques contenant des dérives caféines carboxyliques neutralisés par des bases organiques et préparations utiles dans le traitement de la cellulite.**

(57) La présente invention concerne des compositions topiques hétérogènes à base de microgranules contenant des caféines carboxylates de bases organiques, utiles comme amincissant et/ou dans le traitement de la cellulite, ainsi que leur préparation.

La composition topique hétérogène, selon l'invention, utile comme amincissant et/ou dans le traitement de la cellulite, est caractérisée en ce qu'elle comporte à titre de principes actifs l'acide caféine carboxylique salifié par des bases organiques cosmétologiquement acceptables, lesdits principes actifs étant présents au sein de microgranules et/ou de microparticules en suspension dans un gel hydroalcoolique.

EP 0 371 874 A1

Xerox Copy Centre

# AMINCISSANTS TOPIQUES CONTENANT DES DERIVES CAFEINES CARBOXYLIQUES NEUTRALISES PAR DES BASES ORGANIQUES ET PREPARATIONS UTILES DANS LE TRAITEMENT DE LA CELLULITE.

La présente invention, réalisée au Centre Dermatologique et Cosmétologique Pierre FABRE, concerne des nouvelles formulations cosmétiques hétérogènes contenant des principes actifs amincissants utiles dans le traitement de la cellulite.

Les principes actifs sont maintenus en suspension au sein d'un gel hydroalcoolique, sous la forme de microgranules ou de microparticules.

Selon la présente invention, les principes actifs utilisés dans les formulations sont des dérivés de l'acide caféine carboxylique de formule I :

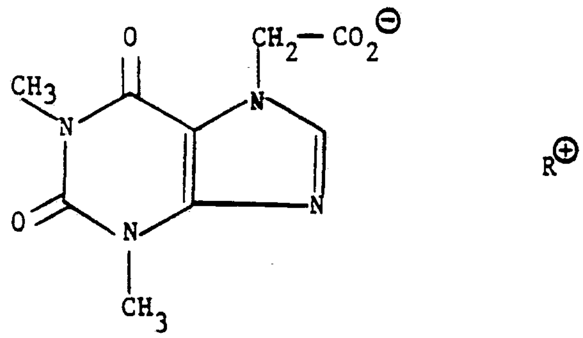

(I)

dans laquelle :
R⊕ représente des bases organiques protonées et plus particulièrement les bases organiques suivantes :

$N(CH_2 - CH_2\ OH)_3$ ; 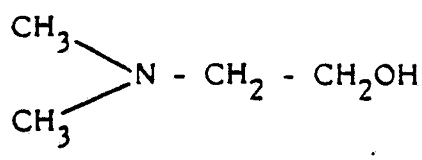

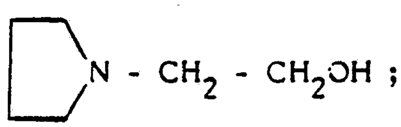 ; 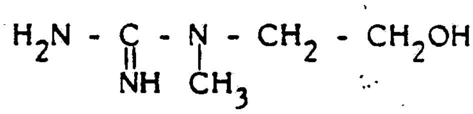 et

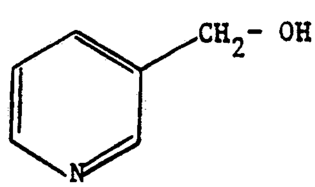

Les principes actifs selon la présente invention ont été soumis à des tests pharmacocinétiques en utilisant l'acide caféine carboxylique marqué au carbone 14.

Les résultats de cette étude montrent que la vitesse de passage transcutané est plus rapide quand l'agent salifiant est une base organique et plus particulièrement pour le caféine carboxylate de 3 nicotinol, ce qui confirme la supériorité de cet actif dans le traitement de la cellulite. Les principes actifs de la présente invention peuvent également être utilisés en association avec la caféine, la vitamine E et/ou ses dérivés.

La préparation des nouveaux principes actifs utilisés dans la présente invention est illustrée par les exemples suivants :

EXEMPLE 1

Préparation de caféine carboxylate de 3 nicotinol

Ce composé chimique peut être obtenu par le procédé suivant : 1 mole de 3 nicotinol (109,1 g) est ajouté à 1 mole d'acide caféine carboxylique (238,2g) que l'on dilue dans 3 litres d'eau, le mélange réactionnel est chauffé jusqu'à dissolution. La solution est concentrée jusqu'à siccité, on récupère quantitativement le produit de formule

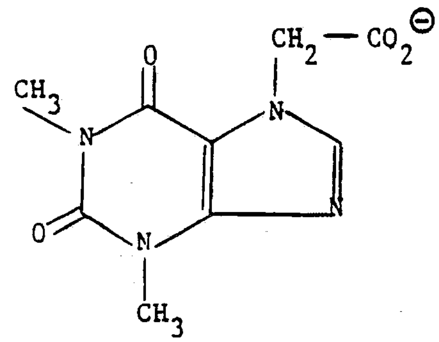

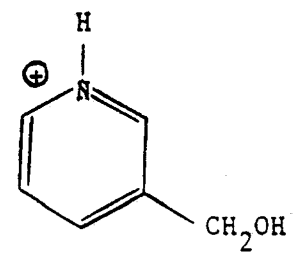

Formule brute :

$C_{15}H_{17}O_5N_5$ PM : 347,34

Caractéristiques physico-chimiques :

Cristaux blancs. Point de fusion : 145° C

| Analyse élémentaire (lot MEI25) | | | |
|---|---|---|---|
| | C | H | N |
| Calculée | 51,87% | 4,95% | 20,16% |
| Trouvée | 51,90% | 4,88% | 20,15% |

Solubilité :

2% dans l'eau.

Le produit peut également s'obtenir pour des quantités industrielles par atomisation à partir d'une solution aqueuse. Il faut noter que compte tenu de l'entraînement à la vapeur du 3 nicotinol il faut opérer sous pression réduite et dans une fourchette étroite de température.

D'une manière similaire à celle décrite dans l'exemple 1, les produits suivants ont été préparés.

EXEMPLE 2

Caféine carboxylate de triéthanolamine

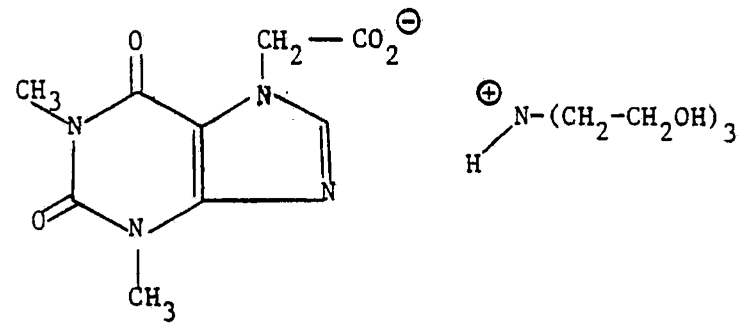

<u>Formule brute</u> :

$C_{15}H_{25}N_5O_7$ PM : 387,4

<u>Caractéristiques physico-chimiques</u> :

Cristaux blancs. Point de fusion : 125° C

| Analyse élémentaire (lot FII6) | | | |
|---|---|---|---|
| | C | H | N |
| Calculée<br>Trouvée | 46,51%<br>46,38% | 6,50%<br>5,85% | 18,08%<br>19,24% |

<u>Solubilité</u> :

0,4% dans l'eau.

<u>EXEMPLE 3</u>

<u>Caféine carboxylate de β diméthylaminoéthanol</u>

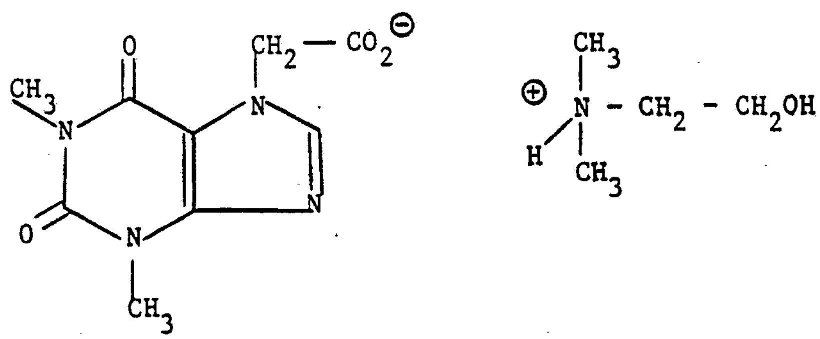

<u>Formule brute</u> :

$C_{13}H_{21}N_5O_5$ PM : 327,34

<u>Caractéristiques physico-chimiques</u> :

Cristaux blancs. Point de fusion : 120° C

| Analyse élémentaire (lot FII7) | | | |
|---|---|---|---|
| | C | H | N |
| Calculée<br>Trouvée | 47,70%<br>47,40% | 6,47%<br>6,03% | 21,40%<br>21,52% |

Solubilité :

0,8% dans l'eau.

EXEMPLE 4

Caféine carboxylate de β pyrrolidino éthanol

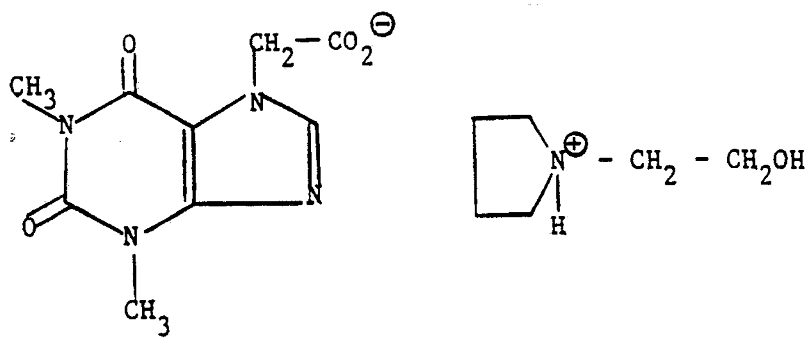

Formule brute :

$C_{15}H_{23}N_5O_5$ PM : 353,38

Caractéristiques physico-chimiques :

Cristaux blancs. Point de fusion : 126° C

| Analyse élémentaire (lot FII8) | | | |
|---|---|---|---|
| | C | H | N |
| Calculée<br>Trouvée | 50,98%<br>51,01% | 6,56%<br>6,50% | 19,82%<br>20,02% |

Solubilité :

0,5% dans l'eau.

EXEMPLE 5

Caféine carboxylate de créatinol

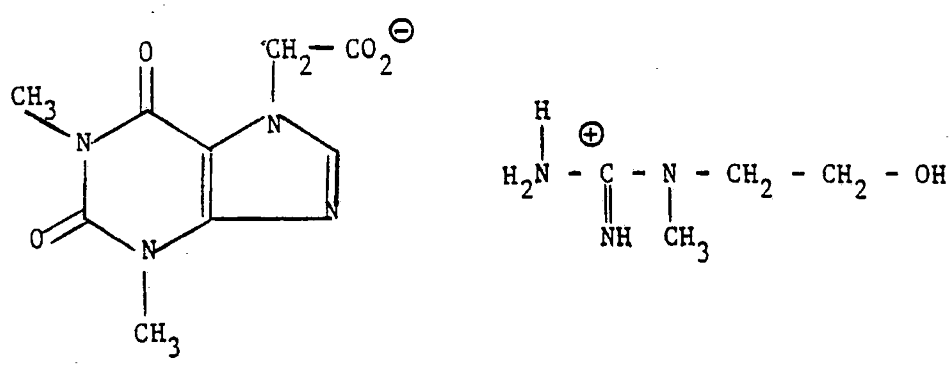

Formule brute :

$C_{13}H_{21}N_7O_5$ PM : 355,36

Caractéristiques physico-chimiques :

Cristaux blancs. Point de fusion : 190° C.

| Analyse élémentaire (lot AXXI 53) | | | |
|---|---|---|---|
| | C | H | N |
| Calculée | 43,94% | 5,95% | 27,59% |
| Trouvée | 44,68% | 5,70% | 27,83% |

Solubilité :

12% dans l'eau.

Selon la présente invention, les préparations galéniques hétérogènes à usage cosmétique contiennent comme principes actifs des caféines carboxylates de formule générale II, associées ou non à des dérivés de la vitamine E et/ou à la caféine.

Ces principes actifs sont libérés de façon progressive et prolongée lors de l'application topique.

Une telle libération prolongée est obtenue grâce à la préparation de particules hétérogènes comportant au moins deux phases solides.

La première phase comporte un support liant inerte thermoplastique et la second phase comporte des particules imprégnées de principes actifs. Le procédé de préparation de telles formulations comporte les étapes suivantes :

1. La première phase est micronisée jusqu'à l'obtention de lamelles de taille moyenne inférieure à 50µm.

2. Les particules de charge imprégnées d'actifs sont micronisées jusqu'à l'obtention de particules de taille moyenne inférieure à 50µm.

3. Les lamelles obtenues au cours de l'étape 1 sont mélangées avec les particules obtenues au cours de l'étape 2.

4. L'éthanol est ajouté comme agent mouillant, après séchage et ventilation, on récupère une poudre qui est calibrée par tamisage pour obtenir une taille de particule entre 0,2 et 2mm.

Les particules hétérogènes chargées d'actifs microgranulés selon la présente invention sont de préférence :

des principes actifs à visée amincissante et/ou utiles dans le traitement de la cellulite et principalement : le caféine carboxylate de 3 nicotinol, le caféine carboxylate de triéthanolamine, le caféine carboxylate de

diméthylaminoéthanol, le caféine carboxylate de β pyrrolidino éthanol, le caféine carboxylate de créatinol, éventuellement associé à :

. des vitamines et plus particulièrement la vitamine E et/ou ses dérivés.

. des dérivés xanthiniques et plus particulièrement la caféine.

Les formulations comportent de 0,5 à 20% P/P de microgranules chargées d'actifs dans une base appropriée contenant des excipients cosmétiques connus de l'homme de l'art : cette base peut également contenir des actifs selon l'invention.

A titre d'exemples non limitatifs sont citées les formulations des microgranules présentes dans les gels hydroalcooliques amincissants.

| Exemple 1 | |
|---|---|
| Caféine carboxylate de 3 nicotinol<br>Amidon<br>Ethylcellulose<br>Autres excipients | 5 à 20%<br>20 à 30%<br>5 à 20%<br>qsp 100% |
| Exemple 2 | |
| Caféine carboxylate de triéthanolamine<br>Amidon<br>Autres excipients | 10 à 40%<br>20 à 30%<br>qsp 100% |
| Exemple 3 | |
| Caféine carboxylate de diméthylaminoéthanol<br>Caféine<br>Vitamine E<br>Excipients | 10 à 40%<br>5 à 10%<br>10 à 20%<br>qsp 100% |
| Exemple 4 | |
| Caféine carboxylate de β pyrrolodino éthanol<br>Vitamine E<br>Excipient | 10 à 40%<br>5 à 20%<br>qsp 100% |
| Exemple 5 | |
| Caféine carboxylate de créatinol<br>Amidon<br>Autres excipients | 5 à 30%<br>5 à 20%<br>qsp 100% |

Les formulations selon la présente invention sont bien tolérées, elles mettent en jeu des microparticules molles ne faisant intervenir aucun agent minéral susceptible d'être considéré comme corps étranger. Selon la présente invention, les formulations préparées, compte-tenu de la présence de principes actifs tels que les caféines carboxylates d'amines organiques possèdent d'excellentes propriétés amincissantes et se sont révélées très efficaces dans le traitement de la cellulite.

**Revendications**

1. Composition topique hétérogène, utile comme amincissant et/ou dans le traitement de la cellulite, caractérisée en ce qu'elle comporte à titre de principes actifs des sels organiques de l'acide caféine carboxylique, lesdits principes actifs étant présents en solution et/ou sous la forme de microgranules en suspension au sein d'un gel hydroalcoolique.

2. Composition selon la revendication 1, caractérisée en ce que lesdites compositions contiennent en outre de la vitamine E et/ou de la caféine et/ou l'un de ses dérivés.

3. Composition selon l'une des revendications 1 et 2 caractérisées en ce que les dérivés de l'acide caféine carboxylique répondent à la formule générale (I)

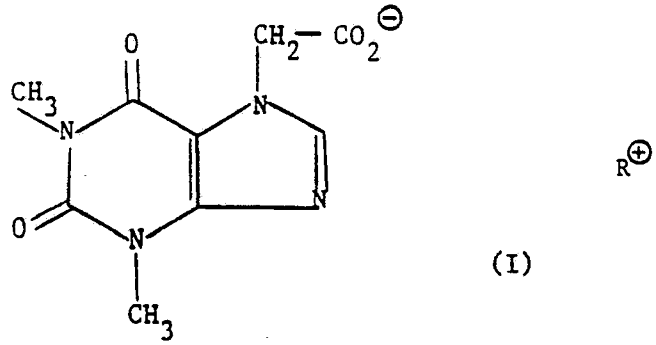

(I)

dans laquelle :
R⊕ représente les bases organiques protonées cosmétologiquement acceptables.

4. Composition selon la revendication 3, caractérisée en ce que le dérivé de l'acide caféine carboxylique est plus particulièrement le caféine carboxylate de 3 nicotinol.

5. Composition selon la revendication 3, caractérisée en ce que le dérivé de l'acide caféine carboxylique est plus particulièrement le caféine carboxylate de triéthanolamine.

6. Composition selon la revendication 3, caractérisée en ce que le dérivé de l'acide caféine carboxylique est plus particulièrement le caféine carboxylate de β diméthylaminoéthanol.

7. Composition selon la revendication 3, caractérisée en ce que le dérivé de l'acide caféine carboxylique est plus particulièrement le caféine carboxylate de β pyrrolidino éthanol.

8. Composition selon la revendication 3, caractérisée en ce que le dérivé de l'acide caféine carboxylique est plus particulièrement le caféine carboxylate de créatinol.

9. Procédé de préparation de microgranules contenant des actifs selon l'une des revendications 1 à 8, caractérisé en ce que la préparation des microgranules comprend les étapes suivantes :

a) La première phase comportant un support liant inerte et micronisé jusqu'à l'obtention de lamelles de taille moyenne inférieure à 50µm.

b) Les particules de charge imprégnées d'actifs sont micronisées jusqu'à l'obtention de particules de taille moyenne inférieure à 50µm.

c) Les lamelles obtenues au cours de l'étape a) sont mélangées avec les particules obtenues au cours de l'étape b).

d) L'éthanol est ajouté comme agent mouillant, après séchage. La poudre obtenue est tamisée pour former des microgranules entre 0,2 et 2 mm de diamètre.

Office européen des brevets

# RAPPORT DE RECHERCHE EUROPEENNE

Numero de la demande

EP 89 40 3293

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | FR-A-2 554 344 (PF COSMETIQUE)<br>* Page 2, lignes 9-31; page 3, lignes 7-19; page 4, lignes 15-28 * | 1-9 | A 61 K 7/48<br>C 07 D 473/08 |
| Y | FR-A-2 267 783 (PIERRE FABRE S.A.)<br>* Page 1, lignes 1-13; page 2, lignes 31-39; revendications 1-4 * | 1-4 | |
| Y | FR-M- 4 298 (A. BUZAS)<br>* Page 1, colonne de gauche, lignes 1-12 * | 5 | |
| Y | CHEMICAL ABSTRACTS, vol. 53, no. 1, 10 janvier 1959, colonnes 382-383, Columbus, Ohio, US; G. SERCHI et al.: "1,3-Dimethylxanthine-7-acetic acids. VII. Some alkylamino salts of theophylline-7-acetic acid and of its 8-nitro derivative", & CHIMICA (MILAN) 34, 60-61 (1958)<br>* Colonne 383, lignes 6-12 * | 6 | |
| | | | **DOMAINES TECHNIQUES RECHERCHES (Int. Cl.5)** |
| Y | DE-A-2 416 556 (HENKEL & CIE GmbH)<br>* Page 1, lignes 1-3; page 3, paragraph 2 * | 7 | A 61 K<br>C 07 D |
| Y | FR-A-2 248 032 (PIERRE FABRE S.A.)<br>* Page 1, lignes 1-9; page 5, tableau, formule 6; revendications 1,9 * | 8 | |
| Y | FR-A-2 611 497 (PIERRE FABRE COSMETIQUE)<br>* Revendications 1,9,11,12 *<br>--- -/- | 9 | |

Le présent rapport a été établi pour toutes les revendications

| Lieu de la recherche | Date d'achèvement de la recherche | Examinateur |
|---|---|---|
| LA HAYE | 23-02-1990 | MUELLNERS W. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen des brevets

# RAPPORT DE RECHERCHE EUROPEENNE

Numero de la demande

EP 89 40 3293

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A-2 490 492 (RIKER LABORATORIES INC.)<br>* Page 1, paragraphe 4 - page 2, ligne 2; exemples 1,2 *<br>--- | 1-9 | |
| A | DE-A-2 436 467 (HENKEL & CIE GmbH)<br>* Page 1, lignes 1-3; page 3, paragraphes 2,3 *<br>----- | 1-9 | |
| | | | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| LA HAYE | 23-02-1990 | MUELLNERS W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................
& : membre de la même famille, document correspondant

EPO FORM 1503 03.82 (P0402)